# EUROPEAN PATENT APPLICATION

(11) **EP 0 663 403 A1**
(43) Date of publication of application: **19.07.1995**
(21) Application number: 94203361.4
(22) Date of filing: 18.11.1994
(51) Int. Cl.: C07K 14/06, C12N 15/38, A61K 39/265, C12N 7/01

(54) **Vector vaccines of bovine herpesvirus I**

(30) Priority: 23.11.1993 EP 93203274
(71) Applicant: Akzo Nobel N.V., NL-6824 BM Arnhem (NL)
(72) Inventor: Keil, Günther, D-72076 Tübingen (DE)
(74) Representative: Mestrom, Joannes Jozef Louis

(57) **Abstract**

The present invention is concerned with a Bovine Herpesvirus I (BHV-I) mutant comprising a heterologous gene introduced into a section of the BHV-I genome. The invention also relates to a vector vaccine comprising such a BHV-I mutant which expresses a heterologous polypeptide derived from a bovine pathogen and induces an adequate immune response in an inoculated host against both FHV an the bovine pathogen. Furthermore, the invention relates to a method for obtaining a BHV-I mutant by self-selection.

SEQUENCE LISTING

(1) GENERAL INFORMATION:

(i) APPLICANT:
(A) NAME: AKZO NOBEL N.V.
(B) STREET: Velperweg 76
(C) CITY: Arnhem
(E) COUNTRY: The Netherlands
(F) POSTAL CODE (ZIP): 6824BM

(ii) TITLE OF INVENTION: Recombinant Bovine Herpesvirus vaccine

(iii) NUMBER OF SEQUENCES: 2

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(2) INFORMATION FOR SEQ ID NO: 1:

(i) SEQUENCE CHARACTERISTICS:
(A) LENGTH: 1289 base pairs
(B) TYPE: nucleic acid
(C) STRANDEDNESS: double
(D) TOPOLOGY: linear

(ii) MOLECULE TYPE: DNA (genomic)

(iii) HYPOTHETICAL: NO

(iii) ANTI-SENSE: NO

(vi) ORIGINAL SOURCE:
(A) ORGANISM: Bovine herpesvirus

(ix) FEATURE:
(A) NAME/KEY: CDS
(B) LOCATION: 142..1095
(D) OTHER INFORMATION: /product= "Protein"
/standard_name= "ORF-1"

(ix) FEATURE:
(A) NAME/KEY: misc_RNA
(B) LOCATION: 1..3
(D) OTHER INFORMATION: /function= "stop-codon gIV"

(ix) FEATURE:
(A) NAME/KEY: misc_RNA
(B) LOCATION: 4..141
(D) OTHER INFORMATION: /function= "untranslated region"

(xi) SEQUENCE DESCRIPTION: SEQ ID NO: 1:

TGAGCGGCCT AGGCCCTCCC CCGACCGCCC CCTTTGCTCC TAGCCCCCGG CTCCAGCCGA 60

GCCGCGCGGG GCGGGAGATA AAGCGCCCGC GCGGCGGCGA CTCAGGCCAT TGCCGCGACC 120

TTGTCCTCCG GCGCGCTCGC G ATG CGG CGC CTG TTG CTC TGG ATG GTG GTG 171
Met Arg Arg Leu Leu Leu Trp Met Val Val
1               5                   10

CTG GCC GCG CGA GCG GCG CCC GCT CGC AGC CTT GTG TAT CGC GGC GAG 219
Leu Ala Ala Arg Ala Ala Pro Ala Arg Ser Leu Val Tyr Arg Gly Glu
                15                  20                  25

GCA GTC GGC CTG CGC GCG GAC GGC CCC GTA GCG TTC GCT GTC CAC CCG 267
Ala Val Gly Leu Arg Ala Asp Gly Pro Val Ala Phe Ala Val His Pro
            30                  35                  40

ACC GAC GCA ACG CTC GCG CTG CGG GGC CGG CTG ATT TTC CTA GAA CAC 315
Thr Asp Ala Thr Leu Ala Leu Arg Gly Arg Leu Ile Phe Leu Glu His
        45                  50                  55

CAG CTC CCG GCC GGG CGG CGC TAC AAC GGG ACC GTC GAG CTG CTG CGC 363
Gln Leu Pro Ala Gly Arg Arg Tyr Asn Gly Thr Val Glu Leu Leu Arg
    60                  65                  70

TAC CAC GCC GCG GGC GAC TGC TTC GTT ATG CTG CAG ACG ACC GCG TTC 411
Tyr His Ala Ala Gly Asp Cys Phe Val Met Leu Gln Thr Thr Ala Phe
75                  80                  85                  90

GCC TCC TGC CCG CGC GTC GCG AAC GAC GCC TTT CGC TCC TGC CTG CAC 459
Ala Ser Cys Pro Arg Val Ala Asn Asp Ala Phe Arg Ser Cys Leu His
                95                  100                 105

GCC GAC ACG CGC CCC GCT CGC AGC GAG CGG CGC GCG AGC GCC GCG GTC 507
Ala Asp Thr Arg Pro Ala Arg Ser Glu Arg Arg Ala Ser Ala Ala Val
            110                 115                 120

GAA AAC CAC GTG CTC TTC TCC ATC GCC CGT CCG CGC CCA ATA GAC TCG 555
Glu Asn His Val Leu Phe Ser Ile Ala Arg Pro Arg Pro Ile Asp Ser
        125                 130                 135

GGG CTC TAC TTT CTG CGC GTC GGC ATC TAC GGC GGC ACC GCG GGC AGC 603
Gly Leu Tyr Phe Leu Arg Val Gly Ile Tyr Gly Gly Thr Ala Gly Ser
    140                 145                 150

GAG CGC CGC CGA GAC GTC TTT CCC TTG GCC GCG TTT GTA CAC AGC TTC 651
Glu Arg Arg Arg Asp Val Phe Pro Leu Ala Ala Phe Val His Ser Phe
155                 160                 165                 170

GGT GAG CCC GGA GAC CCA GAG GCC GCG GCC GCG CAC CCC GGC ACC GTC 699
Gly Glu Pro Gly Asp Pro Glu Ala Ala Ala Ala His Pro Gly Thr Val
                175                 180                 185

GAG GCA GTC GAG GCC CGC TGC GAG CGG GGC CTC GAC GCC AGC TCG GCG 747
Glu Ala Val Glu Ala Arg Cys Glu Arg Gly Leu Asp Ala Ser Ser Ala
            190                 195                 200

AGC CTC TAC GAC CGC GCG CTG GCG GCG TTC CCC GCA GGC GCC GCC ACC 795
Ser Leu Tyr Asp Arg Ala Leu Ala Ala Phe Pro Ala Gly Ala Ala Thr
        205                 210                 215

ACG CCC GGC CCC ACC GCG AGC AGC GAG AGC GGG GCC GCG ACG CCA GAG 843
Thr Pro Gly Pro Thr Ala Ser Ser Glu Ser Gly Ala Ala Thr Pro Glu
    220                 225                 230

AGG GTC GAC GAG ACG ACG GAA GTC GAG GCC GCG ACG AGA GCG GGC TCG 891
Arg Val Asp Glu Thr Thr Glu Val Glu Ala Ala Thr Arg Ala Gly Ser
235                 240                 245                 250

GCG TTT GCC CTC ACC ACG CCC CCG GCC GGC CCG ACC GCC AGC CCC GCC 939
Ala Phe Ala Leu Thr Thr Pro Pro Ala Gly Pro Thr Ala Ser Pro Ala
                255                 260                 265

GCC AGC CCC TCC CGT GCC TTT AGC GCG GCC GCC CCG GCC GCC GCT GCG 987
Ala Ser Pro Ser Arg Ala Phe Ser Ala Ala Ala Pro Ala Ala Ala Ala
            270                 275                 280

CAG CCG GCC GGA GAC ACG CCC GCT CGC TTC CGG CGC CAA CTG GCG TCG 1035
Gln Pro Ala Gly Asp Thr Pro Ala Arg Phe Arg Arg Gln Leu Ala Ser
        285                 290                 295

ATC CTA GTG CCT CTG TGC GTG CTG GTG CTG CTG CTG CTG CGC TCT GCG 1083
Ile Leu Val Pro Leu Cys Val Leu Val Leu Leu Leu Leu Arg Ser Ala
    300                 305                 310

CCG CGA CGG TAAACTGCGC GCTGCGCCGT CGCCTGCTGC CGTGCTCTCG 1132
Pro Arg Arg
315

GCGCGTTTAC AAGCCGCGGA CGTGCGCGGC GTGCGGGAGC GGCACTGCGC GGGGCGGCCC 1192

CCTGCCGCGG CGCGGCACCG AGCGCCCCAG CCACCGTCGT GGCACTGGGC TCCCGGCCAA 1252

GGCGCCCCCC CTCGCCACCA TCAGCGAAGA ATAAATT 1289

(2) INFORMATION FOR SEQ ID NO: 2:

(i) SEQUENCE CHARACTERISTICS:
(A) LENGTH: 317 amino acids
(B) TYPE: amino acid
(D) TOPOLOGY: linear

(ii) MOLECULE TYPE: protein

(xi) SEQUENCE DESCRIPTION: SEQ ID NO: 2:

Met Arg Arg Leu Leu Leu Trp Met Val Val Leu Ala Ala Arg Ala Ala
1               5                   10                  15

Pro Ala Arg Ser Leu Val Tyr Arg Gly Glu Ala Val Gly Leu Arg Ala
            20                  25                  30

Asp Gly Pro Val Ala Phe Ala Val His Pro Thr Asp Ala Thr Leu Ala
        35                  40                  45

Leu Arg Gly Arg Leu Ile Phe Leu Glu His Gln Leu Pro Ala Gly Arg
    50                  55                  60

Arg Tyr Asn Gly Thr Val Glu Leu Leu Arg Tyr His Ala Ala Gly Asp
65                  70                  75                  80

Cys Phe Val Met Leu Gln Thr Thr Ala Phe Ala Ser Cys Pro Arg Val
                85                  90                  95

Ala Asn Asp Ala Phe Arg Ser Cys Leu His Ala Asp Thr Arg Pro Ala
            100                 105                 110

Arg Ser Glu Arg Arg Ala Ser Ala Ala Val Glu Asn His Val Leu Phe
        115                 120                 125

Ser Ile Ala Arg Pro Arg Pro Ile Asp Ser Gly Leu Tyr Phe Leu Arg
    130                 135                 140

Val Gly Ile Tyr Gly Gly Thr Ala Gly Ser Glu Arg Arg Arg Asp Val
145                 150                 155                 160

Phe Pro Leu Ala Ala Phe Val His Ser Phe Gly Glu Pro Gly Asp Pro
                165                 170                 175

Glu Ala Ala Ala Ala His Pro Gly Thr Val Glu Ala Val Glu Ala Arg
            180                 185                 190

Cys Glu Arg Gly Leu Asp Ala Ser Ser Ala Ser Leu Tyr Asp Arg Ala
        195                 200                 205

Leu Ala Ala Phe Pro Ala Gly Ala Ala Thr Thr Pro Gly Pro Thr Ala
    210                 215                 220

Ser Ser Glu Ser Gly Ala Ala Thr Pro Glu Arg Val Asp Glu Thr Thr
225                 230                 235                 240

Glu Val Glu Ala Ala Thr Arg Ala Gly Ser Ala Phe Ala Leu Thr Thr
                245                 250                 255

Pro Pro Ala Gly Pro Thr Ala Ser Pro Ala Ala Ser Pro Ser Arg Ala
            260                 265                 270

Phe Ser Ala Ala Ala Pro Ala Ala Ala Ala Gln Pro Ala Gly Asp Thr
        275                 280                 285

Pro Ala Arg Phe Arg Arg Gln Leu Ala Ser Ile Leu Val Pro Leu Cys

    290                 295                 300

Val Leu Val Leu Leu Leu Leu Arg Ser Ala Pro Arg Arg
305                 310                 315

## Description

The present invention relates to a Bovine Herpesvirus I mutant comprising a mutation in a section of the BHV-genome, such a BHV-I mutant comprising a heterologous DNA sequence inserted in said section, a nucleic acid sequence comprising at least part of said section of the BHV-I genome, a recombinant DNA molecule comprising heterologous DNA flanked by DNA derived of said section, a host cell transfected with said nucleic acid sequence or recombinant DNA molecule, a host cell infected with a BHV-I mutant, a vaccine comprising a BHV-mutant, a method for the immunization of animals against an infectious disease and a method for the preparation of a BHV-I mutant.

The herpesviruses are a widespread family of large viruses containing double-stranded DNA, causing disease in mammals, birds, reptiles, fish and amphibians.
The double-stranded DNA codes for about 30-35 polypeptides, depending on the genus. The molecular weight of the DNA greatly varies between 120 kbp in Channel Cat Fish Herpesvirus to 230 kbp in Human Cytomegalovirus. The GC-content of the DNA varies even more; from 32% in Dog Herpesvirus to 72-73% in Bovine Herpesvirus.

The viral agents causing Infectious Bovine Rhinotracheitis (IBR), Infectious Pustular Vulvovaginitis (IPV) and Infectious Balanoposthitis (IBP) belong to the Herpesviruses, more specifically to the sub-family Alphaherpesvirinae (Roizman, B. In Fields, B.N., Knipe, D.M. (ed.) Fundamental Virology. Ravens Press, New York: 841-847 (1991)).
They are all designated Bovine Herpesvirus I (Roizman et al.; Intervirology 16: 210-217 (1981)), and more recently Bovid Herpesvirus I (Ludwig, H. in B.Roizman (Ed.) The Herpesviruses. Plenum Press New York, vol. 2: 135-214 (1983)). In cross-neutralization tests BHV-I isolates all exhibit only one serotype, regardless their origin from IBR or IPV/IBP cases (Gillespie et al.; Cornell Vet. 49: 288-297 (1959), McKercher et al.; Can. J. Comp. Med. 23: 320-328 (1959)). Restriction enzyme analysis of large numbers of different isolates has revealed differences in restriction site patterns, but no correlation exists between the patterns and the origin of the isolates. Therefore it is now generally accepted that the site of infection with BHV-I determines the effects of the disease, whereas the causative agent is in all cases the Bovine Herpesvirus I.

Transmission of Infectious Bovine Rhinotracheitis can occur for instance a) by animals that are acutely infected with or without showing clinical signs, and that are still shedding virus, and b) by animals that are latently infected and start shedding recurrent virus after stress.
Spread of IPV/IBP occurs e.g. by contaminated semen, natural service, the teaser bull, or by the herdsmen.

The most common manifestation of BHV-I infection is bovine rhinotracheitis which varies from a mild respiratory disease to a severe infection of the entire respiratory tract.
From an economical point of view, IBR is also the most dramatic manifestation of BHV-I infection.
Another relatively important form of BHV-I infection is the genital form, leading to pustular vulvovaginitis in cows and, comparatively, to infectious balanoposthitis in bulls.
Although seen less frequently, the virus is also associated with abortion, conjunctivitis and encephalitis.

BHV-I is widely spread among cattle in all continents, but its host range is limited. Many wild species have been found seropositive, but distinct clinical signs have only been observed in cattle.
Although IBR and IPV have been known in Europe for more than a century, it appears that wild ruminants in Africa and in zoos are the true reservoir of the virus.

Morbidity rates in both IBR and IPV are usually 100% provided that the animals are in close contact (for IPV).
If morbidity is < 100% then the animals are protected by antibodies. Fatalities are rare however, in the case of IBR and IPV. In young calves however, death as a result of BHV-I induced encephalitis is common. Next to this, several strains of BHV-I have shown to induce abortion.
Due to secondary bacterial infections, pneumoniae and enteritis may occur (Bielefeld-Ohmann et al.; J. Infect. Diseases 151: 937-947 (1985))

Immunity against Herpesviruses is depending on at least two mechanisms: a) the induction of neutralizing antibodies and complement-dependent lysis for the inactivation of free virus, and b) the induction of cytotoxic T-cells for the elimination of virus-infected cells.
At present, in general, cattle can be protected against infection by these pathogenic micro-organisms with live vaccines, inactivated vaccines and subunit vaccines.
However these types of vaccines may suffer from a number of drawbacks. The use of attenuated vaccines always carries the risk of inoculation of animals with inadequately attenuated pathogenic micro-organisms. In addition, the attenuated micro-organisms may revert to a virulent state resulting in disease of the inoculated animals and, consequently, spread of the pathogens to other animals. Moreover, a problem with combined live viral vaccines is the mutual influence of the antigenic components resulting in a decrease of potency of one or more of the components.
Inactivated vaccines are generally considered safe, but they generally induce only a low level of immunity, requiring repeated immunizations. Furthermore, the neutralization inducing antigenic determinants of the pathogens may become altered by the inactivation treatment, decreasing the immunising potency of the vaccine. In addition, cellular immunity is to a lesser extend triggered by inactivated vaccines.
Subunit vaccines have the same drawbacks as inactivated vaccines, and additionally, their spectrum of protection is smaller since they contain less different antigenic determinants then the whole organism.
It is an object of the present invention to provide a safe live BHV-1 mutant which can be used not only for the preparation of a vaccine against Bovine Herpesvirus I, but also as a carrier of genetic information for antigenic determinants of other infectious diseases of cattle, which obviates any potential risk associated with the use of a live attenuated pathogen. Therefore the present invention is concerned with a vaccine, which stimulates both the cellular and humoral immune system in a potent way without the explicit need of an adjuvant and which offers the possibility of a multivalent vaccine without the risk of adverse mutual interference of different antigenic components.

The present invention provides a BHV-I mutant comprising a mutation in a section of a BHV-I genome, which is located adjacent to the 3' terminus of gIV and extending to the 3'-terminus of the adjacent ORF-1, i.e. the open reading frame encoding the polypeptide shown in SEQ ID NO: 2., and additionally comprising a poly-adenylation signal which is located between the 3' terminus of the gIV gene and the mutation.

A mutation is understood to be a change of the genetic information in the above-mentioned section in comparison to the genetic information present in this section of the genome of the parent BHV-I.

The mutation may be a nucleic acid substitution, deletion, insertion or inversion, or a combination thereof. In particular the mutation is an insertion, preferrably of a heterologous gene.

Preferrably, the mutation is incorporated into a gene encoding the polypeptide shown in SEQ ID NO: 2. (mentioned ORF-1 hereafter) or in the intergenic region between the gene encoding gIV and the gene encoding the polypeptide shown in SEQ ID NO: 2.

More in particular, the mutation is incorporated in the nucleic acid sequence shown in SEQ ID NO: 1.

The term "polypeptide" as used herein refers to a molecular chain of amino acids. "Polypeptide" does not refer to a specific length of the product. The polypeptide can if required be modified in vivo or in vitro, for example by glycosylation, amidation, carboxylation, myristylation or phosphorylation; thus inter alia, peptides, oligopeptides, and proteins, as well as modifications thereof, are included within the definition of polypeptide.
A poly-adenylation signal is a short sequence at the 3' terminus of RNA, which in most cases comprises a consensus sequence AAUAAA, is and encoded by the DNA-fragment from which the RNA was transcribed. Nascent RNA is cleaved at this signal by a specific endonuclease.

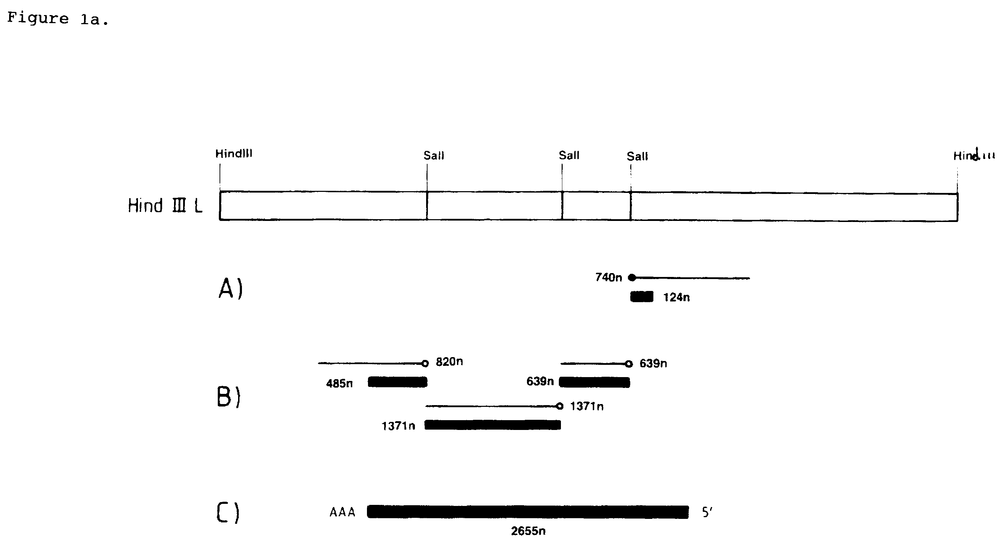

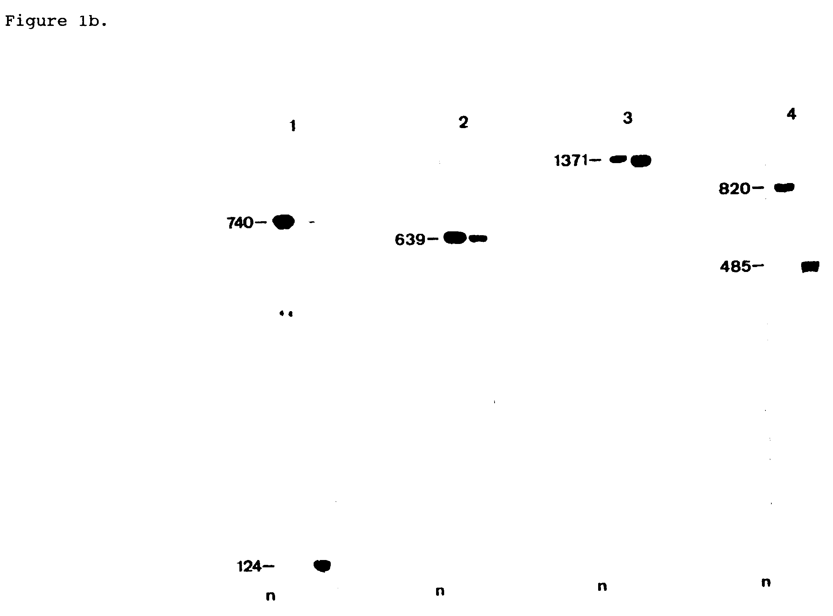

Subsequently, a poly-A polymerase adds a long stretch (usually about 250) A-residues to the RNA beginning at the cleavage site.
No poly-adenylation signal has been found between the gIV gene and the gene coding for ORF-1. It has been shown by RNA-mapping, that the RNAs transcribed from the gIV gene and from the ORF-1 gene both use the poly-A signal downstream ORF-1, as follows: To determine the 5'- and 3' ends of the gIV mRNA, 5'- and 3'- end labeled DNA fragments from the genomic region spanning the gIV and ORF-1 gene were hybridised to RNA from BHV-1 infected cells and subsequently incubated with nuclease S1.
Protected fragments were separated on sequencing gels and visualized by autoradiography. Sizes were determined using end-labeled pBR322 HpaII DNA fragments. The results showed that the mRNA coding for gIV is initiated 25 nucleotides downstream the gIV promotor and terminates 45 nucleotides downstream the poly-A signal which follows ORF-1.
No 3' RNA-discontinuity was detected between the 5'-cap site and the 3'-poly-A signal determined above. Thus the mRNA coding for gIV terminates at the polyadenylation signal downstream ORF-1, i.e. the same signal where the mRNA for ORF-1 is polyadenylated (see Example 1 and figure 1a, b).
In addition to this, it was experimentally shown, that it is impossible to rescue gIV-negative BHV-1 mutants lacking the poly-A signal downstream ORF-1, with a plasmid that contains the gIV coding sequence but no downstream poly-A signal.
The above mentioned results implicate that the RNA coding for the gIV gene product is relatively long. Therefore, modifications in the region between the gIV-gene and the poly-A signal may lead to a non-functional gIV RNA. Clearly, the deletion of the poly-A signal downstream ORF-1 would lead to a non-functional RNA. In order to circumvent this problem, in the present invention a poly-A signal was introduced between the gIV gene and the section indicated in the invention, in which mutations can be made. As a result, the gIV RNA is always provided with a poly-A signal.

Many poly-A signals have been sequenced, and they all share the consensus sequence mentioned above. The sequence of the surrounding nucleotides, the so-called context, may however highly differ from one gene to another. Thus heterologous polyadenylation signals can equally well be used downstream a certain gene, instead of the authentic poly-A signal. In the present invention, the poly-adenylation signal of the murine cytomegalovirus (MCMV) immediate early 2 (ie2) gene (Messerle et al.; J. Virol 65: 1638 ff. (1991)) was used as a heterologous poly-A signal. Other poly-A signals are e.g. the late poly-A signal of the Simian virus 40 (Schek et al., Mol. Cell. Biol. 12: 5386-5393 (1992)), a poly-A signal of HIV-1 (Valsamakis et al., Mol. Cell. Biol. 12: 3699-3705 (1992)) and the poly-A signal of the Bovine parathyriod hormone messenger (Weaver et al., Mol. Cell. Endocr. 28: 411-424 (1982)).

An intergenic region is a nucleic acid sequence that is generally found in between two coding regions. It may contain sequences that are involved in transcription initiation or termination, translation initiation, and the like.

It is possible that some amino acids in the polypeptide encoded by ORF-1 have been replaced by others while the polypeptide is not altered in its function. For instance, a polypeptide containing Asp at a certain site, and its variant containing Asn at the comparable site still have the same properties. This process in which an amino acid is replaced by an functionally analogous amino-acid is called functional displacement.
In this case the variant proteins are called functional variants.
Another cause of variation is the phenomenon of degeneracy of the genetic code. Shortly, it means, that e.g. the amino acid glutamic acid is coded for by both GAT and GAA. This phenomenon holds for all amino acids, except Met and Trp. Thus, it is obvious, that the ORF-1 polypeptide given in the present invention can not only be coded for by the nucleotide sequence given in SEQ ID NO: 1 but also by a very large variety of other sequences, still all giving the same polypeptides.
Therefore, a variant BHV-1 sequence encoding a polypeptide that is functionally comparable to the ORF-1 polypeptide of the present invention can also be used in the invention.

Since there is little selective presure on intergenic sequences, it is possible that variant BHV-1 strains can be found, that have an intergenic region between gIV-ORF and ORF-1 with a slightly different sequence. These variants do belong to the invention.

The prerequisite for a useful BHV-I mutant according to the present invention is, that the mutation is introduced into a permissive position or region of the genomic BHV-I sequence, i.e. a position or region which can be used for the incorporation of the mutation without disrupting essential functions of BHV-I such as those necessary for infection or replication.
Although the general genomic organization of Bovine Herpesvirus with respect to e.g. length, location of Herpesvirus-characteristic repeats and unique regions has been determined (Ludwig, H. in: "The Herpesviruses", Vol. 2, B. Roizman ed., Plenum Press (1983)), knowledge of gene function, gene products and even the exact number of genes, is lacking. Only a few genes are well-studied: the Thymidine Kinase gene (Kit et al., U.S.patent 4.703.011 and 4.824.667) and the gX gene (gX is the nomenclature used for Pseudorabies-virus) (Kit et al., the Veterinary Record 127: 363-365 (1990) and European Patent EP 0.326.127). The gX-homolog gene gG from BHV-I has recently been localized and sequenced by Cochran et al. (PCT-application WO 93/02104). Also four major BHV-I glycoproteins gI, gII, gIII and gIV have been described in the literature (Fitzpatrick et al. J. of Virol. 62, 4239-4288 (1988), and three of the genes; gI (in fact the gI-homolog gene gE from BHV-I: gI is the PRV-nomenclature) (Whitbeck et al.; J. Virol. 62: 3319-3327 (1988)) ,(Rijsewijk (WO 92/21751)), gIII (Fitzpatrick et al.; Virology 173: 46-57 (1989)) and gIV (Tikoo et al., J. of Virol. 64: 5132-5142 (1990)) have been sequenced. Several of these genes have been found and localized on the basis of the structural or functional homology with their counterparts in Pseudorabies virus and Herpes simplex virus. This could of course only be done for 1) those genes that have sufficient sequence homology or for 2) those genes that code for polypeptides with significant homology to glycoproteins found in Herpes simplex virus and Pseudorabies virus (Fehler et al., J. of Virol., 66: 831-839 (1992)).
This is however only the case for few of the BHV-I genes, as can easily be concluded from e.g. the differences in GC-content or genome-length between various herpesviruses as mentioned above.

The section of the BHV-1 genome referred to in the present invention has been described partially in the literature. The gIV gene has been sequenced by Tikoo (Tikoo et al., J. of Virol. 64: 5132-5142 (1990)). Tikoo only sequenced the coding region, but he assumes the presence of a poly-A signal. This assumption however is not correct since no poly-A consensus-signal was found, as was mentioned above.

It has been surprisingly found now, that the gene coding for ORF-1 as described in the present invention allows mutations of the kind mentioned above, without disrupting information essential for the reproduction of the virus. There is however, as was also found, a certain proviso: expression of the essential gIV gene needs the presence of a poly-A signal at a reasonable distance of the gIV gene. Since gIV uses the poly-A signal of ORF-1, gIV has to be provided with a (homologous or heterologous) poly-A signal located between the gIV gene and the mutation, before mutations can be introduced in ORF-1.

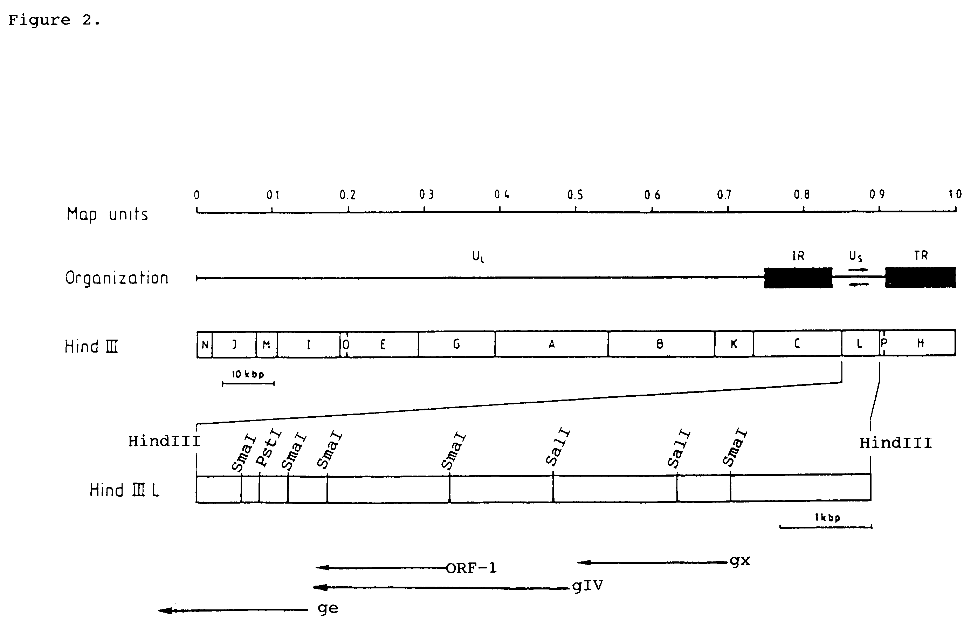

The section of the BHV-I genome used to introduce one or more mutations in order to prepare a BHV-I mutant according to the invention is located within an about 4600 bp. fragment generated by partial digestion of the BHV-I DNA with the restriction enzyme SmaI. See figure 2.

The BHV-I mutant of the present invention thus comprises a mutation in a section of a BHV-I genome, which is located adjacent to the 3' terminus of gIV and extends to the 3' end of the adjacent ORF-1, and additionally comprises a polyadenylation signal which is located between the 3' terminus of the gIV gene and the mutation.

In particular, the BHV-mutant comprises a mutation in a section of a BHV-I genome that spans the DNA fragment of which the sequence is shown in SEQ ID NO: 1. This sequence represents the region comprising the open reading frame ORF-1 as represented by SEQ ID NO: 1 and the intergenic region between ORF-1 and gIV.

Also part of the invention is a BHV-I mutant comprising a heterologous DNA sequence in the above mentioned section.
A heterologous DNA sequence is a sequence that originates from a source, other than the parent BHV-I strain. It may be derived from the DNA of another organism or may be synthetically made. Also included is cDNA, made from heterologous RNA or heterologous single-stranded DNA.

The heterologous DNA sequence may be a random sequence or it may be a sequence that interferes with transcription or translation of ORF-1. Such sequences may be transcription termination signals or polyadenylation sites but they may also contain translational stop-codons.

In a preferred form , the heterologous DNA sequence codes for a polypeptide.

The heterologous DNA sequence preferably contains promotor sequences such that expression is under control of these sequences. These sequences may be the promotor sequences that are found to be linked to the gene coding for the polypeptide, in its native form, or it may be other promotor sequences.

It is obvious to those skilled in the art that the choice of a promotor extends to any eukaryotic, prokaryotic, viral or synthetically prepared promotor capable of directing gene transcription in cells infected by the BHV-I mutant, e.g. promotors of the retroviral long terminal repeat (Gorman et al., Proc. Natl. Acad. Sci. USA 79, 6777-6781, 1982), the SV40 promotor (Mulligan and Berg, Science 209, 1422-1427, 1980) or the cytomegalovirus immediate early promotor (Schaffner et al., Cell 41, 521-530, 1985).

Sequences modulating expression such as e.g. enhancers are often found to play an essential role in transcription and transcription-levels, and are therefore also considered to be part of the transcription complex.

In a more preferred form, the heterologous DNA sequence encodes an antigen of a significant cattle-pathogen, which is able to elicit a protective immune response, whereby the antigen is expressed by the BHV-I mutant according to the invention upon replication in the host cell.

Preferably the antigen is chosen from the group of cattle pathogens, consisting of Bovine Rotavirus, Bovine Viral Diarrhoea virus, Parainfluenza type 3 virus, Bovine Paramyxovirus, Foot and Mouth Disease virus, Bovine Coronavirus, Bovine Respiratory Syncytial virus and Pasteurella haemolytica.

Furthermore, the heterologous DNA sequence may encode a cytokine. Several cytokines, e.g. interferons are known to play an important role as immune modulators. Thus it may be advantageous to include genetic information for this kind of molecule into said section.
It is obvious that a heterologous DNA sequence can be introduced at a certain site in said section, e.g. in a restriction site without deleting any nucleotides from the section. On the other hand, it is possible to exchange one or more nucleotides with heterologous DNA sequences of equal or different length.

Therefore, BHV-I mutants having a deletion in said section are also included in the invention, regardless the presence and size of a heterologous DNA sequence.

The technique of in vivo homologous recombination can be used to introduce a modified homologous nucleic acid sequence comprising a mutation in a section of the BHV-I genome as described above, or a heterologous nucleic acid sequence into the BHV-I genome.

Homologous recombination can be accomplished by first constructing a recombinant DNA molecule for recombination with BHV-I genomic DNA. Such a molecule may be derived from any suitable plasmid, cosmid or phage, plasmids being most preferred, and contains the modified homologous or the heterologous DNA sequence, if desired operably linked to a promotor. Said DNA sequence and promotor are introduced into a fragment of genomic BHV-I DNA containing the whole or part of the non-essential section of the BHV-I genome as defined herein, sub-cloned in the recombinant DNA molecule.
These so called insertion-region sequences which flank the heterologous DNA sequence should be of appropriate length, e.g. 50-3000 bp, as to allow in vivo homologous recombination with the viral BHV-I genome to occur.

If desired, a construct can be made which contains two or more different heterologous DNA sequences derived from the same or different pathogens said sequences being flanked by insertion-region sequences of BHV-I defined herein. Such a recombinant DNA molecule can be employed to produce recombinant BHV-I which expresses two or more different antigenic polypeptides to provide a multivalent vaccine.

Thus the present invention also relates to a nucleic acid sequence comprising at least part of the section of the BHV-genome that is located 3' adjacent to the gIV gene and extending to the 3' end of ORF-1.

In a preferred form, a recombinant DNA molecule comprising a heterologous DNA sequence, flanked at its 5'- and/or 3'-terminal site by DNA sequences derived from a section of a BHV-I genome as defined above is used for homologous recombination.

More preferrably, a recombinant molecule is used for homologous recombination, that comprises the nucleic acid sequence or the recombinant DNA molecule described above, and additionally comprises at least a 3'-terminal part of a gene coding for a BHV-1 gIV polypeptide and a poly-adenylation signal adjacent to the 3'-terminus of this gene.

In a most preferred form, the above mentioned recombinant molecule comprises not only part of the gIV gene, but the whole gIV gene, including a promotor functionally linked to the gene. This recombinant molecule is, after homologous recombination, capable of fully complementing a gIV-negative BHV-1 mutant.

Susceptible cells, e.g. Madin Darby Bovine Kidney cells (MDBK-cells) or bovine embryo cells can be transfected with BHV-1 DNA in the presence of the recombinant DNA molecule comprising a heterologous DNA sequence, and also comprising a poly-A signal located upstream the heterologous DNA sequence as defined above, flanked at its 5'- and/or 3'-terminal site by DNA sequences derived from a section of the BHV-1 genome as defined in the present invention whereby recombination occurs between the insertion-region sequences in the recombinant DNA molecule and the insertion-region sequences in the BHV-I genome.

Recombination can also be brought about by first transfecting cells with a nucleic acid sequence containing the modified homologous DNA sequence or heterologous DNA sequence flanked by appropriate insertion-region sequences, followed by infection with BHV-I.

Recombinant viral progeny is thereafter produced in cell culture and can be selected for example genotypically or phenotypically, e.g. by hybridization, detecting enzyme activity encoded by a gene co-integrated along with the heterologous DNA sequence or detecting the antigenic heterologous polypeptide expressed by the recombinant BHV-I immunologically. Recombinant virus can also be selected positively based on resistance to compounds such as neomycine, gentamycine or mycophenolic acid. The selected recombinant BHV-I can be cultured on a large scale in cell culture after which BHV-I mutant-containing material or heterologous polypeptides expressed by said BHV-I mutant can be collected thereof.

As mentioned above, the BHV-1 mutant may also have deletions in the ORF-1 coding region.
One way of introducing deletions is cloning of the BHV-1 section defined above into a plasmid, selective cutting of the BHV-1 section defined above with suitable restriction-enzymes that cut in ORF-1, removal of the fragment that was cut, and recircularisation of the remaining plasmid molecule.
Recircularization would result in a derivative lacking at least part of the coding sequence present within the newly identified region.

Alternatively, progressive deletions can be introduced either in one or two directions starting from within a restriction site present within the sequence of an open reading frame. Enzymes such as Bal31 or exonuclease III can be used for this purpose. Recircularized plasmid molecules are transformed into E. coli cells and individual colonies are analyzed by restriction mapping in order to determine the size of the deletion introduced into the specified region. An accurate positioning of the deletion can be obtained by sequence analysis. The plasmid containing a defined deletion can be cotransfected with BHV-I viral DNA into cultured bovine cells. After in vivo recombination has occurred, the deletion will be introduced at the correct position within the described region of the viral genome.
Recombinants among the viral progeny can be identified for example by means of 15 to 20 bases long synthetic oligomer which hybridizes specifically to the nucleotide sequence which is generated at the junction where the deletion originally was introduced.

A live BHV-I mutant according to the present invention, and in particular a live BHV-I expressing one or more different heterologous polypeptides of specific pathogens, can be used to vaccinate cattle. Vaccination with such a live vector vaccine is preferably followed by replication of the BHV-I mutant within the inoculated host, expressing in vivo the heterologous polypeptide along with the BHV-I polypeptides. The polypeptides expressed in the inoculated host will then elicit an immune response against both BHV-I and the specific pathogen. If the heterologous polypeptide derived from the specific pathogen can stimulate a protective immune response, then the animal inoculated with the BHV-I mutant according to the invention will be immune to subsequent infection by that pathogen as well as to infection by BHV-I. Thus, a heterologous nucleic acid sequence incorporated into the insertion-region of the BHV-I genome according to the invention may be continuously expressed in vivo, providing a solid, safe and long-lasting immunity to a pathogen.

A BHV-I mutant according to the invention containing and expressing one or more different heterologous polypeptides can serve as a monovalent or multivalent vaccine.

For the preparation of a live vaccine the BHV-I mutant according to the present invention can be grown on susceptible cells.

Growth can e.g. be performed on a cell culture of bovine origin.

The viruses thus grown can be harvested by collecting the tissue cell culture fluids and/or cells. The live vaccine may be prepared in the form of a suspension or may be lyophilized.

In addition to an immunogenically effective amount of the recombinant BHV-I the vaccine may contain a pharmaceutically acceptable carrier or diluent.

Examples of pharmaceutically acceptable carriers or diluents useful in the present invention include stabilizers such as SPGA, carbohydrates (e.g. sorbitol, mannitol, starch, sucrose, glucose, dextran), proteins such as albumin or casein, protein containing agents such as bovine serum or skimmed milk and buffers (e.g. phosphate buffer).
Optionally, one or more compounds having adjuvant activity may be added to the vaccine. Suitable adjuvants are for example aluminium hydroxide, phosphate or oxide, oil-emulsions (e.g. of Bayol F^{(R)} or Marcol 52^{(R)}, saponins or vitamin-E solubilisate.

The useful effective amount to be administered will vary depending on the age, weight, mode of administration and type of pathogen against which vaccination is sought. A suitable dosage can be for example about 10³ - 10⁷ pfu/animal.

An BHV-I mutant according to the invention can also be used to prepare an inactivated vaccine.

For administration to animals, the BHV-I mutant according to the present invention can be given inter alia intranasally, intradermally, subcutaneously or intramuscularly.
It is a further object of the present invention to produce subunit vaccines, pharmaceutical and diagnostic preparations comprising a heterologous polypeptide expressed by an BHV-I mutant according to the invention.
This can be achieved by culturing cells infected with said BHV-I under conditions that promote expression of the heterologous polypeptide. The heterologous polypeptide may then be purified with conventional techniques to a certain extent depending on its intended use and processed further into a preparation with immunizing, therapeutic or diagnostic activity.

The above described active immunization against specific pathogens will be applied as a protective treatment in healthy animals. It goes without saying that animals already infected with a specific pathogen can be treated with antiserum comprising antibodies evoked by a BHV-I mutant according to the invention comprising a heterologous gene derived from the specific pathogen encoding an antigenic polypeptide. Antiserum directed against a recombinant BHV-I according to the invention can be prepared by immunizing animals, for example cattle, with an effective amount of said BHV-I mutant in order to elicit an appropriate immune response. Thereafter the animals are bled and antiserum can be prepared.

Homologous recombination is occurring only at a very low frequency. Therefore, selection of a recombinant BHV-I virus generally is very time-consuming.
The present invention offers a method to avoid selection for homologous recombinants, by using a self-selecting system. In general, the fact that a gene is essential for a virus implicates that no virus can be grown that is deprived of the genetic information to express the functional gene product unless the cell complements for the missing gene product.
This implicates, that it is impossible to grow infectious virus that is genotypically negative for the essential polypeptide gIV, unless this virus is grown on a gIV-complementing cell-line.
In the present invention, it is shown for the first time, how this phenomenon can be used to avoid time-consuming selection procedures as follows: A virus, genotypically negative for an essential gene can only be grown in cells that are continuously complementing the missing gene-product. In that case, infectious virus will emerge, that is capable of infecting non-complementing cells, but is not capable of reproduction.
When however the DNA of the virus is transfected into a non-complementing cell together with a recombinant DNA having homologous DNA sequences flanking an intact copy of the gene missing in the virus, this may lead to homologous recombination restoring the essential gene function in the viral genome.
As a result, this virus will be the only virus that, by regaining its essential information, is capable of reproduction on non-complementing cells. Therefore the process is self-selective.

Therefore, in a further form of the invention, a method is used for the construction of a BHV-I mutant comprising bringing together in a suitable host cell a recombinant DNA molecule comprising the genetic information for functional expression of the gIV polypeptide said genetic information being flanked at its 5'- and 3'-terminal site by DNA sequences derived from a section of a BHV-I genome that comprises a gene or an intergenic region, and a genotypically gIV-negative BHV-1 virus or the DNA of such a genotypically gIV-negative BHV-1 virus.

The section of the BHV genome may comprise an essential gene or a non-essential gene. This depends on the aim of the experiment. If the goal is to inactivate an essential gene, flanking sequences could be derived from this essential gene. Recombinant viruses will then lack the functional essential gene. If the goal is, to merely insert foreign sequences, a non-essential gene or a non-essential intergenic region will usually be used to provide the flanking sequences.

In a more preferred form, a method will be used in which the genetic information also comprises a heterologous gene.
Such a heterologous gene may be derived, e.g. a bovine pathogen, e.g. from the group consisting of Bovine Rotavirus, Bovine Viral Diarrhoea virus, Parainfluenza type 3 virus, Bovine Paramyxovirus, Foot and Mouth Disease virus, Bovine Coronavirus, Bovine Respiratory Syncytial virus and Pasteurella haemolytica.

Transfection of the recombinant DNA molecule together with DNA of said virus, that lacks the genetic information to produce a functional gIV polypeptide leads after homologous recombination to a viable virus carrying both the necessary genetic information for gIV and the desired mutation, e.g. the inserted genetic information for a foreign antigen. In a variation on this method, cells are transfected with the recombinant DNA molecule and infected with the virus that lacks the genetic information to produce a functional gIV polypeptide.

In an even more preferred form of the invention, a method is used, that comprises bringing together in a suitable host cell a recombinant DNA molecule that comprises a section of the BHV-1 mutant according to the present invention and at least a 3'-terminal part of the gene coding for gIV and a poly-A signal adjacent to the 3'-terminus of this gene, and a genotypically gIV-negative BHV-I virus from which the 3'-terminal genetic information of the gIV gene present in the recombinant DNA molecule or less is deleted, or the DNA of such a genotypically gIV-negative BHV-1 virus.

In the present invention, preferably a heterologous poly-A signal is chosen as signal 3' adjacent to the gIV gene for the following reason: homologous recombination may occur between:
a) a gIV-negative BHV-1 mutant comprising regions upstream the gIV gene and in or downstream of ORF-1, that are homologous to those in a plasmid, and
b) a plasmid comprising one of these homologous regions located upstream of gIV, followed by the gIV gene, a poly-A signal downstream gIV-ORF, a mutation, if necessary a poly-A signal downstream of the mutation, and finally the other homologous region.
If as a poly-A signal 3' terminal of gIV the authentic gIV-poly-A signal is used, it is possible that the typical 3' site at which homologous recombination crossover occurs, comprises the context of the poly-A signal. In that case, all sequences downstream of the poly-A site would not be transferred to the BHV-1 virus during the recombination event. As a result, the mutation as described in the present invention, e.g. the heterologous gene would not be transferred to the mutant virus.
Insertion of a heterologous poly-A signal 3' adjacent to the gIV gene and insertion of the authentic poly-A signal downstream of the mutation solves this problem: no recombination between heterologous poly-A signals can happen since the poly-A consensus-sequence is statistically too short to allow homologous recombination. Thus, if 3' homologous recombination occurs at the poly-A signal, this can only be the authentic poly-A signal and therefore all genetic information in between this signal and the 5' homologous recombination site is also transferred.

It is not necessary that the whole gIV gene is present on the recombinant DNA molecule if the viral DNA still comprises the part of the gIV gene missing on the recombinant DNA molecule and an overlapping gIV fragment: if an overlapping sequence for homologous recombination is located in the gIV genome, recombination leads to a full-length gene.

The advantage of the method for the production of BHV-1 mutants described above, is that the only viable progeny viruses are viruses that successfully passed homologous recombination.
This avoids the laborious and timeconsuming screening procedures that normally follow homologous recombination experiments.

For homologous recombination, a BHV-I mutant virus is used, that has a mutation in the part of gIV that is incorporated in the recombinant DNA molecule or less than that part, and as a result is not capable of expressing functional gIV polypeptide. This mutant is grown on a gIV complementing cell line as described by Fehler et al. (Fehler et al., J. of Virol., 66: 831-839 (1992)). As a result, only BHV-I viruses that recombined successfully obtained the genetic information for expression of a functional gIV polypeptide. Thus, only these viruses are capable of multiple replication in non-complementing host cells. The method therefore provides an extremely efficient and simple way of screening for successful homologous recombination events.

The usefulness of the method for the highly efficient production of BHV-1 mutants as described above is not limited to the combination of the non-essential region ORF-1 as disclosed in the present application with the gIV gene.
A recombinant DNA molecule comprising the gene coding for gIV, including promotor and poly-adenylation sites, i.e. the genetic information for functional expression of the gIV polypeptide which genetic information is additionally flanked at its 3'- and 5' ends by fragments from any gene or intergenic region of BHV-1 can be used in combination with a gIV-negative BHV-1 virus for homologous recombination. Homologous recombination then leads to complementation of the gIV-gene, albeit not at its native site.
This method may e.g. be used to introduce an insertion in a gene in order to inactivate this gene. Interesting gene candidates for this approach of insertion-inactivation are e.g. genes, involved in virulence.

Preferrably, the genetic information mentioned above is extended with additional genetic information. This additional information may be in the form of a heterologous gene.

Although not necessarily, in many cases a gene or intergenic region known to be non-essential in BHV-I will be chosen as the preferred insertion site for gIV.

In a preferred form, a recombinant DNA molecule comprising the gene coding for gIV, including promotor and poly-adenylation sites, and additionally comprising a heterologous gene, which recombinant DNA molecule is additionally flanked at its 3'- and 5' ends by fragments from any gene or intergenic region known to be non-essential in BHV-I is used.

The gI-gene (Whitbeck et al.; J. Virol. 62: 3319-3327 (1988)), the gIII-gene (Fitzpatrick et al.; Virology 173: 46-57 (1989)) or the TK-gene (Kit et al., U.S.Patent 4.703.011 and 4.824.667) as mentioned above and all three known to be non-essential genes could for example be used as a possible integration site for a gIV/foreign gene construct, analogous to the principles described above for ORF-1.

### Example 1.

### Localisation of the gIV-poly-A signal.

In order to demonstrate that the poly-A signal used by gIV is the same signal used by ORF-1, the folowing experiment was done: the HindIII L fragment was cleaved with SalI and fragments containing gIV and ORF-1 were labeled with ³²P at the 5'-end (figure 1a, closed circle) or 3'-end (figure 1a, open circle). Labeled fragments were hybridized to RNA from BHV-1 infected cells and, after nuclease S1 digestion, separated on denaturing acrylamide gels. Labeled fragments were visualized by autoradiography. The size of the protected fragments was determined using sequencing reactions and end-labeled DNA markers; sizes of the probes were determined from the sequence. The results show, that the infected cell RNA protects 124 nucleotides (n) of the 740 n 5'-end labeled fragment (A in figure 1a, lane 1 in figure 1b), the full lengths of the 3'-labeled 639 n and 1371 n fragments (figure 1b, lanes 2 and 3) and 485 n of the 3'-labeled 820 n fragment (figure 1b, lane 4). This demonstrates that the 5'-end of the gIV mRNA is 124 n upstream of the rightward SalI cleavage site (25 n downstream the TATA-box, and 485 n downstream the leftward SalI cleavage site (45 n downstream the poly-A signal). No RNA discontinuity was found in between. Thus the transcript coding for gIV spans 2655 nucleotides of the genomic sequence (C) and contains both the gIV-ORF and ORF-1.

### Example 2.

### Construction of a plasmid carrying the gIV gene including promotor and termination signals.

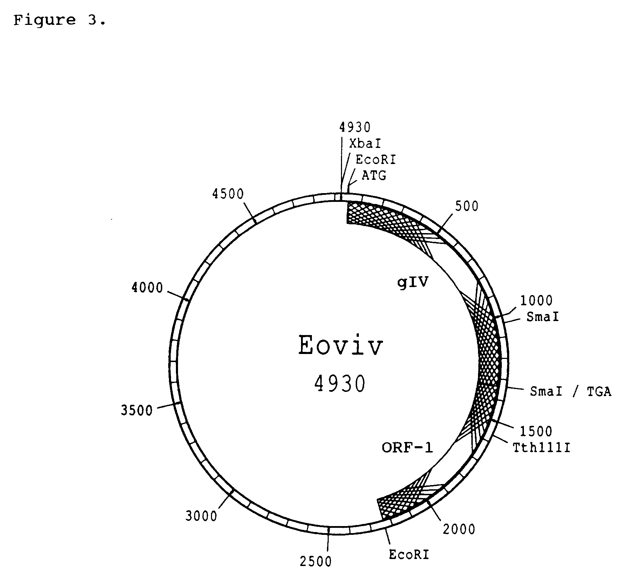

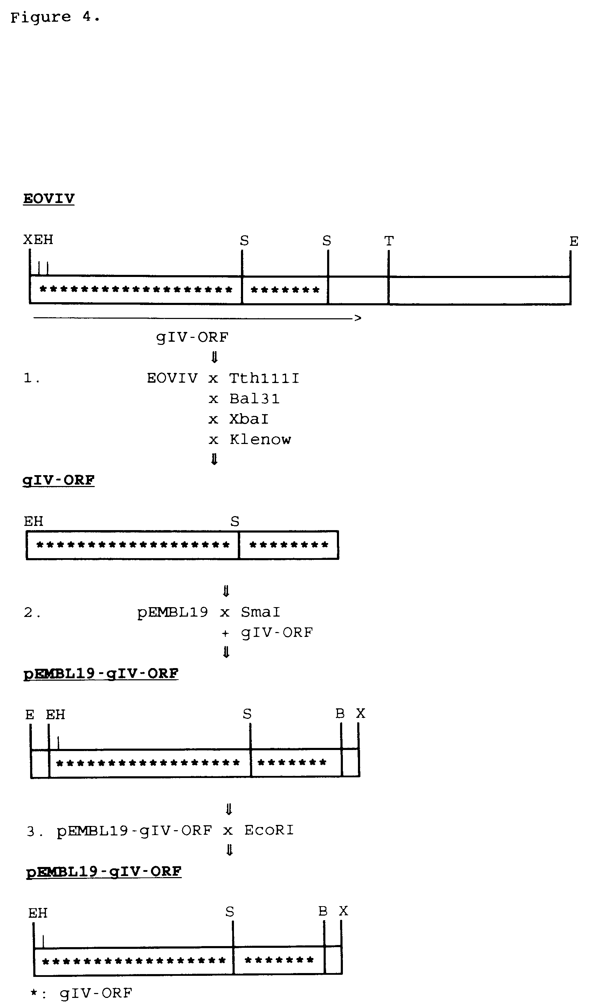

The first step in constructing this plasmid was the isolation of the gIV ORF from the plasmid Eoviv (figure 3). This plasmid contains a 2,2 kbp fragment carrying the gIV-ORF and ORF-1. It was incubated with Tth111I, which cleaves within ORF-1 (see figure 4). To remove ORF-1 specific sequences the cleaved DNA was incubated with exonuclease Bal31 followed by cleavage with XbaI, which cuts upstream the translational start codon for gIV, and recessive ends were filled with the Klenow fragment of E. coli DNA-polymerase I (Klenow-polymerase). The reaction products were size separated in agarose gels and DNA fragments of the expected size were isolated and integrated into the plasmid vector pEMBL19^{(R)}, linearized with SmaI. DNA of resulting clones was sequenced to determine the extend of the ORF-1 specific sequence deletion. One clone, lacking the entire ORF-1 and 12 nucleotides of the 3'-end of the gIV ORF was cleaved with EcoRI and religated. This step was necessary because due to the cloning procedure this plasmid contained several restriction enzyme cleavage sites 5' to the gIV-ORF flanked by EcoRI cleavage sites.
The resulting plasmid was named pEMBL19-gIV-ORF (see figure 4).

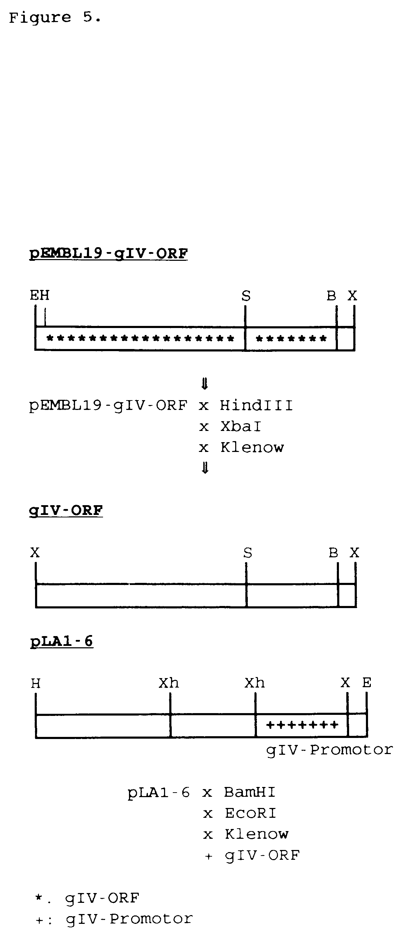

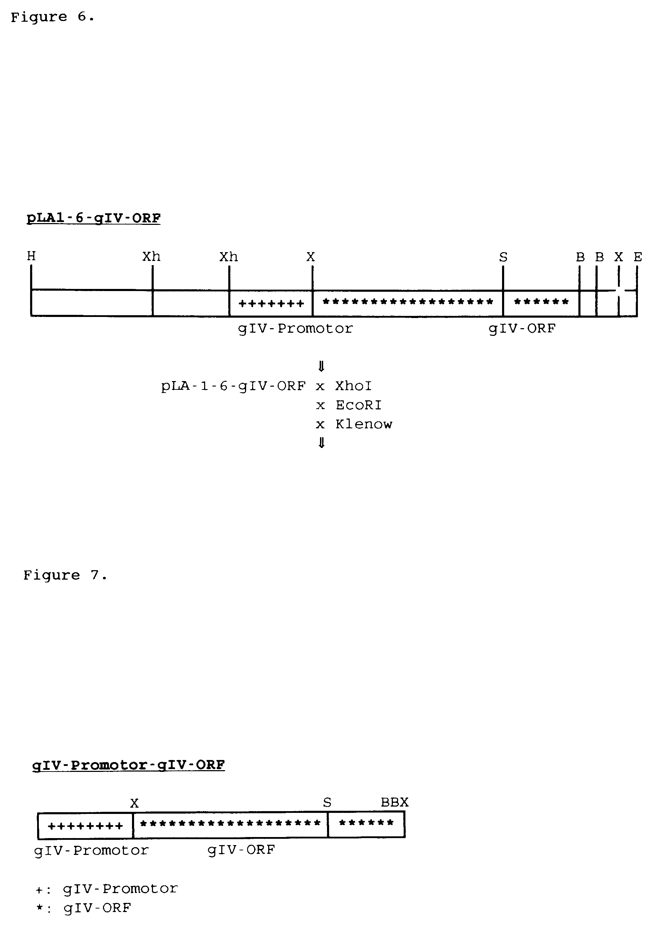

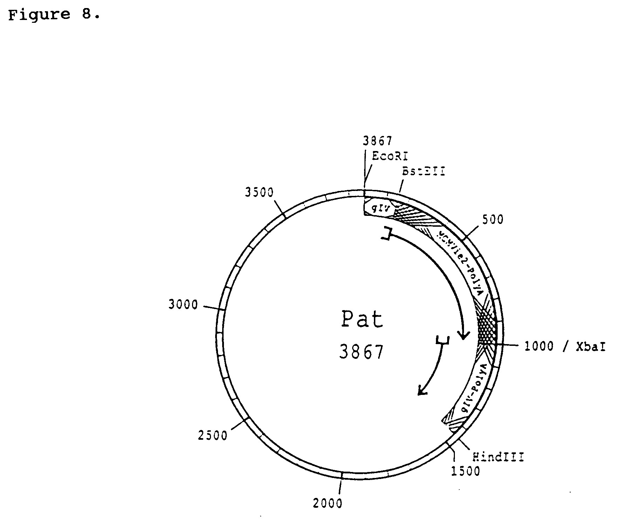

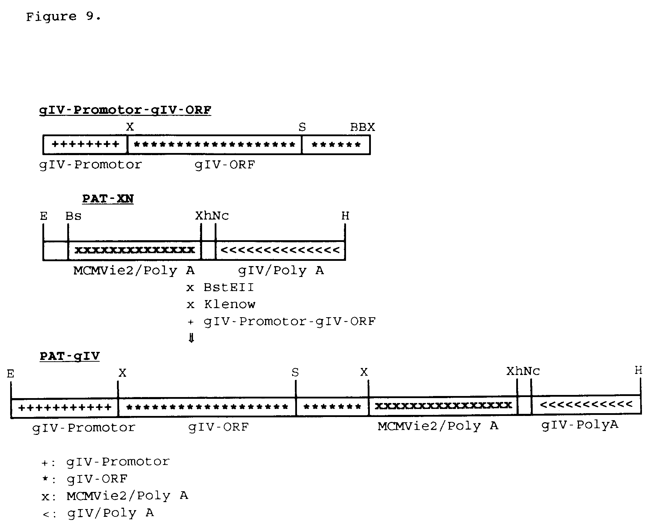

To provide the gIV-ORF with the authentic gIV promoter and with additional sequences upstream the gIV gene for the foreseen integration into the BHV-1 genome, pEMBL19-gIV DNA was cleaved with HindIII (upstream the ORF) and XbaI at the truncated 3' end of the gIV-ORF and recessive ends were filled in with Klenow-polymerase (see figure 5). The DNA fragment was integrated into plasmid pLA1-6, cleaved with BamHI and EcoRI and blunt-ended with Klenow-polymerase. Plasmid pLA1-6 contains the gIV promoter followed by a polylinker sequence and sequences upstream of the gIV gene. The plasmid with the correct orientation of the (still 3' truncated) gIV-ORF was named pLA1-6-gIV. To complete the gIV-ORF and to add a molecular biological marker a synthetic oligonucleotide was inserted into the BamHI cleavage site adjacent to the truncated gIV-ORF. The resulting plasmid that now contained the complete gIV-ORF was named pLA1-6-gIV-ORF (see figure 6).
To construct a complete gIV-gene and a recombination plasmid suitable for the deletion of ORF-1 out to the BHV-1 genome, plasmid DNA of pLA1-6-gIV-ORF was cleaved with XhoI and EcoRI, blunt-ended with Klenow-polymerase and a DNA fragment containing upstream gIV sequences, gIV-promoter and the restored gIV-ORF (see figure 7) was inserted into plasmid pAT-XN treated with BstEII and Klenow-polymerase. Plasmid pAT-XN is based on Pat 3867 that contains the poly-adenylation signal of the murine cytomegalovirus (MCMV) immediate early 2 (ie2) gene (Messerle et al.; J. Virol 65: 1638 ff. (1991)) adjacent to the BstEII site (see figure 8). The MCMV sequences are followed by cleavage sites for XhoI and NcoI and downstream gIV sequences required for homologous recombination into the BHV-1 genome. This was accomplished by inserting the oligonucleotide sequence CTCGAGCCATGG into the XbaI site in Pat 3867. The resulting plasmid was named pAT-gIV and used for the deletion of ORF-1 (see figure 9).
To further demonstrate that the addition of a heterologous polyadenylation signal enables also the integration of additional genes in this region of the BHV-1 genome, plasmid DNA of pAT-gIV was cleaved with NcoI and blunt ended with Klenow-polymerase. The ORF for the bacterial enzyme chloramphenicol acetyltransferase under control of the MCMV early 1 (e1) promoter (Bühler et al.; J. Virol. 64: 1907 ff. (1990)) was integrated into the linearized pAT-gIV DNA, giving the plasmid pAT-gIV CAT.

### Example 3

### Deletion of ORF-1 from the BHV-1 genome.

MDBK cells were transfected with 5 µg DNA of pAT-gIV 24 h after seeding using the calcium phosphate precipitation technique. Transfected cells were shocked with glycerol 4 h after addition of the DNA and cultures were infected with phenotypically complemented gIV⁻ virus BHV-1/80-221 (Fehler et al., J. of Virol., 66: 831-839 (1992)) with a multiplicity of 10 PFU per cell. 48 h p.i. the cells were harvested and the cell culture supernatant was used for the infection of MDBK cells. Under these conditions only genotypically gIV positive virions can grow productively and form plaques on the monolayers. 3 days p.i. virions from single plaques were isolated and DNA from MDBK cells was purified, cleaved with HindIII, size separated by agarose gel electrophoresis and transferred to nitrocellulose filters. Filters were hybridised with ³²P-labeled DNA either from the MCMV-ie2 polyadenylation signal, the β-galactosidase-ORF, ORF-1 or the sequences downstream ORF-1 presented for homologous recombination. The results demonstrate that the isolated virions lack the β-galactosidase sequences, that ORF-1 is deleted and that the gIV-ORF and the MCMV-ie2 DNA is integrated.
From the size of the hybridizing fragments it is concluded that the integration of pAT-gIV Sequences occurred via homologous recombination. Isolated virions were plaque purified twice to ascertain homologous preparations. The ORF-1⁻ virus was designated BHV-1-ORF-1⁻. The determination of the viral growth characteristics (release and total yield from infected cell cultures) and the adsorption and penetration behaviour of the mutant in comparison to wild type virus showed that the deletion of ORF-1 did not affect these parameters in cell culture.

### Deletion of ORF-1 and integration of the CAT gene.

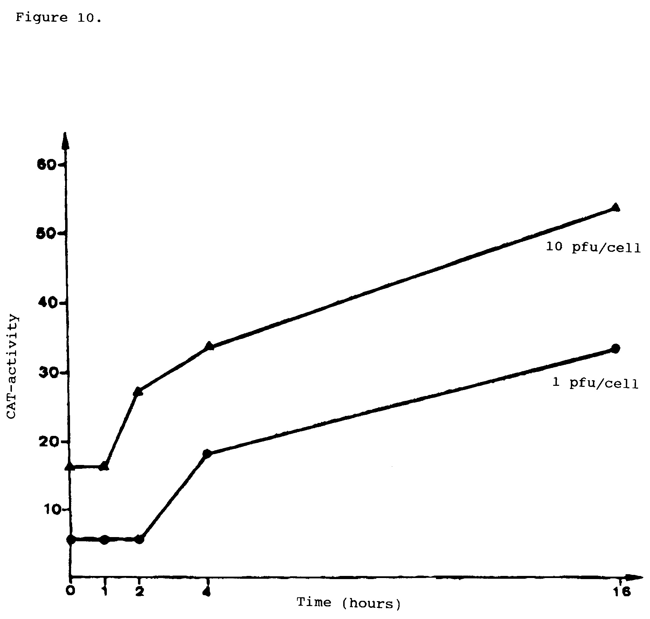

MDBK cells were transfected with 5 µg DNA of pAT-gIVCAT 24 h after seeding using the calcium phosphate precipitation technique. Transfected cells were shocked with glycerol 4 h after addition of the DNA and cultures were infected with phenotypically complemented gIV virus BHV-1/80-221 with a multiplicity of 10 PFU per cell.
48 h p.i. the cells were harvested and the cell culture supernatant was used for the infection of MDBK cells.
Under these conditions only genotypically gIV positive virions can grow productively and form plaques on the monolayers. 3 days p.i. virions from single plaques were isolated and DNA from MDBK cells was purified, cleaved with HindIII, size separated by agarose gel electrophoresis and transferred to nitrocellulose filters. Filters were hybridized with ³²P-labeled DNA either from the CAT-ORF, the β-galactosidase-ORF, ORF-1 or the sequences downstream ORF-1 presented for homologous recombination. The results demonstrate that the isolated virions lack the β-galactosidase sequences, that ORF-1 is deleted and that the gIV-ORF and the MCMVe1-CAT DNA are integrated. From the size of the hybridizing fragments it is concluded that the integration of pAT-gIV sequences occurred via homologous recombination. Isolated virions were plaque purified twice to ascertain homologous preparations. The ORF-1⁻ /CAT⁺virus was designated BHV-1CAT. The determination of the viral growth characteristics (release and total yield from infected cell cultures) and the adsorption and penetration behaviour of the mutant in comparison to wild type virus showed that the replacement of ORF-1 by the CAT gene did not affect these parameters in cell culture.
Additionally, as can be seen from figure 10, expression of the CAT-gene is also unaffected as shown by the production of CAT and the raise in CAT activity after increasing time. There is also a clear correlation between the number of viruses (= plaqueforming units) and CAT production level.

In conclusion: replacement of the ORF-1 gene by the CAT gene does not interfere with absorbtion and penetration behaviour of the virus, and the CAT gene is actively expressed is its new context.

### Legend to the figures.

Figure 1. Schematic representation of nuclease S1 protection experiments (figure 1a) and experimental data (figure b; each panel shows the undigested probe on the left and the protected fragment on the right).
Figure 2. Restriction map of the gIV/ORF-1 region. The arrows indicate the various RNAs.
Figure 3. Physical map of Eoviv.4930.
Figure 4. Construction of pEMBL19-gIV-ORF.
Figure 5. Construction of gIV-ORF/pLA1-6.
Figure 6. Construction of pLA1-6-gIV-ORF.
Figure 7. Construction of gIV-Prom-gIV-ORF.
Figure 8. Physical map of Pat 3867.
Figure 9. Construction of pAT-gIV.
Figure 10. Correlation between CAT-activity and multiplicity of infection, and increase of CAT-activity in time.

## Claims

1. BHV-I mutant comprising a mutation in a section of a BHV-I genome which is located adjacent to the 3' terminus of gIV and extending to the 3'-terminus of the adjacent ORF-1, and additionally comprising a polyadenylation signal which is located between the 3' terminus of the gIV gene and the mutation.

2. BHV-mutant according to claim 1, characterised in that the section spans an open reading frame ORF-1 coding for a polypeptide with a sequence shown in SEQ ID NO: 2 and an intergenic region between the gene encoding the gIV polypeptide and the gene encoding the ORF-1 polypeptide.

3. BHV-mutant according to claim 1 or 2, characterised in that the section spans a DNA fragment with a sequence shown in SEQ ID NO: 1.

4. BHV-I mutant according to claims 1-3, characterised in that the mutation comprises an insertion with a heterologous DNA sequence.

5. BHV-I mutant according to claim 4, characterised in that the heterologous DNA sequence encodes a polypeptide.

6. BHV-I mutant according to claim 5, characterised in that the heterologous DNA sequence encoding a polypeptide is under the control of a promotor regulating the expression of said heterologous DNA sequence in a cell infected with said BHV-I mutant.

7. BHV-I mutant according to claim 5 or 6, characterised in that said heterologous DNA sequence encodes an antigen of a bovine pathogen.

8. BHV-I mutant according to claim 7, characterised in that the antigen is derived from the group consisting of Bovine Rotavirus, Bovine Viral Diarrhoea virus, Parainfluenza type 3 virus, Bovine Paramyxovirus, Foot and Mouth Disease virus, Bovine Coronavirus, Bovine Respiratory Syncytial virus and Pasteurella haemolytica.

9. BHV-I mutant according to claim 5 or 6, characterised in that the heterologous DNA sequence encodes a cytokine.

10. BHV-I mutant according to claims 1-9, characterised in that at least part of the DNA sequence of said section of the BHV-I genome is deleted

11. Nucleic acid sequence comprising at least part of the section of a BHV-I genome defined in claims 1-3.

12. Recombinant DNA molecule comprising a heterologous DNA sequence, flanked at its 5'- and/or 3'-terminal site by DNA sequences derived from a section of a BHV-I genome as defined in claims 1-3.

13. Recombinant DNA molecule comprising the nucleic acid sequence according to claim 11 or the recombinant DNA molecule according to claim 12, and comprising at least a 3'-terminal part of a gene coding for a BHV-1 gIV polypeptide and a poly-adenylation signal adjacent to the 3'-terminus of this gene

14. Recombinant DNA molecule according to claim 13, wherein the gene coding for gIV polypeptide is under the control of a functional promotor.

15. Host cell transfected with a nucleic acid molecule according to claim 12, or a recombinant DNA molecule according to claims 13-14.

16. Host cell infected with a BHV-I mutant according to claims 1-10.

17. Vaccine comprising a BHV-I mutant according to claims 1-10.

18. Method for the immunization of animals against an infectious disease which comprises administering a vaccine according to claim 17.

19. Method for the construction of a BHV-I mutant comprising bringing together in a suitable host cell a recombinant DNA molecule comprising the genetic information for functional expression of the gIV polypeptide said genetic information being flanked at its 5'- and 3'-terminal site by DNA sequences derived from a section of a BHV-I genome that comprises a gene or an intergenic region, and a genotypically gIV-negative BHV-1 virus or the DNA of such a genotypically gIV-negative BHV-1 virus.

20. Method for the construction of a BHV-I mutant according to claim 19, characterised in that the genetic information also comprises a heterologous gene.

21. Method for the construction of a BHV-I mutant according to claims 1-10, comprising bringing together in a suitable host cell the recombinant DNA molecule according to claim 13 or 14, and a genotypically gIV-negative BHV-I virus from which the 3'-terminal genetic information of the gIV gene present in the recombinant DNA molecule or less is deleted, or the DNA of such a genotypically gIV-negative BHV-1 virus.
